# EUROPEAN PATENT APPLICATION

(11) **EP 3 670 533 A1**
(43) Date of publication of application: **24.06.2020**
(21) Application number: 20151513.7
(22) Date of filing: 27.10.2014
(51) Int. Cl.: C07K 16/24, C12N 15/13, C12N 15/63, A61K 39/395, A61P 37/02, A61P 19/02, A61P 25/00, A61P 37/00, A61P 29/00

(54) **IL-17A BINDING AGENT AND USES THEREOF**

(30) Priority: 18.11.2013 CN 201310580942
(62) Divisional of application: 14862443.0
(71) Applicant: Shanghai Hengrui Pharmaceutical Co. Ltd., Minhang District Shanghai 200245 (CN); Jiangsu Hengrui Medicine Co., Ltd., Lianyungang, Jiangsu 222047 (CN)
(72) Inventor: ZHANG, Lianshan, Shanghai, 200245 (CN); LIU, Jiajian, Shanghai, 200245 (CN); CAO, Guoqing, Shanghai, 200245 (CN); SUN, Piaoyang, Jiangsu, 222047 (CN)
(74) Representative: Potter Clarkson

(57) **Abstract**

Provided is an antibody capable of specially recognizing IL-17A and being combined with IL-17A. The antibody can be used for treating inflammations and autoimmune diseases caused by high expression of interleukin-17A, such as psoriasis, psoriatic arthritis, ankylosing spondylitis, multiple sclerosis, inflammatory arthritis.

## Description

### FIELD OF THE INVENTION

The present invention relates to an IL-17A binding agent and its use as a therapeutic agent, in particular as a therapeutic agent for a variety of inflammatory or autoimmune diseases.

### BACKGROUND

Cytokines of interleukin-17 family are named as IL-17A to IL-17F, respectively. At the same time, the family of their receptors, IL-17 receptor A to IL-17 receptor E, is also discovered: These IL-17 cytokines bind to the corresponding receptor, thereby mediate different inflammatory responses.

The most typical member of the family is IL-17A. Lymphocytes which migrate to the infection or injury sites can secrete IL-17A. On one hand, IL-17A induces the expression of inflammatory cytokines and chemokines, thereby recruits more immune cells to the inflammation site and exacerbates inflammatory response; on the other hand, IL-17A induces the expression of some factors relevant to tissue repair, thus accelerates recovery of the organism. Although interleukin-17A has the effect on amplifying immune defense response and protecting organisms during the process of anti-infection and tissue repair in host, in many patients suffering from autoimmune diseases and cancers, interleukin-17A is highly expressed, excessive expression of interleukin- 17A plays a deterioration role in pathologic development, because it can induce the expressions of various inflammatory factors. Many animal experiments have proved that pathological severity of various autoimmune diseases can be effectively suppressed by interleukin-17A deficiency or interleukin-17A antibody neutralization. There is evidence that IL-17 signal showed certain effect as a target for treating autoimmune diseases, including rheumatoid arthritis (RA), psoriasis, Crohn's disease, multiple sclerosis (MS), psoriasis disease, asthma and lupus (see, for example, Aggarwal et al., J.Leukoc.Biol, 71 (1): 1-8 (2002); Lubberts et al.).

Human IL-17 is a gene encoding a polypeptide having up to 155 amino acids. The polypeptide comprises a 19-amino-acid signal sequence and a 132-amino-acid mature area. With relative molecular weight of 17,000Da, human IL-17A is a glycoprotein existing in the form of homodimer or heterodimer (Spriggs et al, J.Clin.Immunol, 17: 366-369 (1997)). IL-17F, a homolog, can combine with IL-17A to form an IL-17A/F heterodimer. The amino acid sequence of IL-17F (IL-24, ML-1) has up to 55% similarity to that of IL-17A, both have the same receptor, IL-17R. IL-17R is ubiquitously expressed in a variety of cells, including vascular endothelial cells, peripheral T cells, B cells, fibroblasts, myelomonocytes and bone marrow stromal cells (Kolls et al, Immunity, 21: 467-476 (2004); Kawaguchi et al, J.Allergy Clin.Immunol, 114 (6): 1267-1273 (2004); Moseley et al, Cytokine Growth Factor Rev, 14 (2): 155-174 (2003)).

From the discovery of interleukin-17A, until now, a variety of anti-IL-17A antibodies have been found, such as CN101001645A, CN101326195A, CN101646690A, but there is still need for developing various kinds of improved antibodies to effectively reduce or eliminate IL-17 activity in inflammatory response and autoimmune diseases.

### SUMMARY OF THE INVENTION

The present invention provides an anti-IL-17A antibody with higher affinity and longer half-life.

The present invention provides an IL-17A binding agent, comprising:
antibody light chain variable region, comprising 0-3 LCDR regions selected from those shown in SEQ ID NO: 13, SEQ ID NO: 14, and SEQ ID NO: 15; and
antibody heavy chain variable region, comprising 0-3 HCDR regions selected from those shown in SEQ ID NO: 10, SEQ ID NO: 11, and SEQ ID NO: 12;
wherein the numbers of CDR regions of antibody light chain variable region and heavy chain variable regions are not simultaneously 0.

According to some embodiments of the present invention, the IL-17A binding agent comprises SEQ ID NO: 13.

According to some embodiments of the present invention, the IL-17A binding agent comprises SEQ ID NO: 14.

According to some embodiments of the present invention, the IL-17A binding agent comprises SEQ ID NO: 15.

According to some embodiments of the present invention, the IL-17A binding agent comprises SEQ ID NO: 10.

According to some embodiments of the present invention, the IL-17A binding agent comprises SEQ ID NO: 11.

According to some embodiments of the present invention, the IL-17A binding agent comprises SEQ ID NO: 12.

According to some embodiments of the present invention, the IL-17A binding agent comprises one LCDR region selected from SEQ ID NO: 13, SEQ ID NO: 14 and SEQ ID NO: 15.

According to some embodiments of the present invention, the IL-17A binding agent, comprises one HCDR region selected from SEQ ID NO: 10, SEQ ID NO: 11 and SEQ ID NO: 12.

According to some embodiments of the present invention, the IL-17A binding agent comprises two LCDR regions selected from SEQ ID NO: 13, SEQ ID NO: 14 and SEQ ID NO: 15.

According to some embodiments of the present invention, the IL-17A binding agent comprises two HCDR regions selected from SEQ ID NO: 10, SEQ ID NO: 11 and SEQ ID NO: 12.

According to some embodiments of the present invention, the IL-17A binding agent comprises three LCDR regions, wherein the amino acid sequence of LCDR1 is shown in SEQ ID NO: 13, the amino acid sequence of LCDR2 is shown in SEQ ID NO: 14, the amino acid sequence of LCDR3 is shown in SEQ ID NO: 15.

According to some embodiments of the present invention, the IL-17A binding agent comprises three HCDR regions, wherein the amino acid sequence of HCDR1 is shown in SEQ ID NO: 10, the amino acid sequence of HCDR2 is shown in SEQ ID NO: 11, the amino acid sequence of HCDR3 is shown in SEQ ID NO: 12.

According to some embodiments of the present invention, the IL-17A binding agent, wherein the antibody light chain variable region further comprises light chain FR region derived from murine κ, λ chain or a variant thereof. In some embodiments, the amino acid sequence of the antibody light chain variable region is SEQ ID NO: 2. Furthermore, the IL-17A binding agent comprises light chain constant region derived from murine κ, λ chain or a variant thereof.

According to some embodiments of the present invention, the IL-17A binding agent, wherein the antibody heavy chain variable region further comprises heavy chain FR region derived from murine IgG1, IgG2, IgG3, IgG4 or a variant thereof. In some embodiments, the amino acid sequence of antibody heavy chain variable region is SEQ ID NO: 1. Furthermore, the IL-17A binding agent comprises heavy chain constant region derived from murine IgG1, IgG2, IgG3, IgG4 or a variant thereof,.

According to some embodiments of the present invention, the IL-17A binding agent, wherein the antibody light chain variable region thereof further comprises light chain FR region derived from human κ, λ chain or a variant thereof; In some embodiments, the light chain FR region of the antibody light chain variable region is human germline light chain A10 FR region whose amino acid is shown in SEQ ID NO: 4, or a variant thereof. In some embodiments, the variant of antibody light chain variable region FR region refers to human germline light chain A10 FR region with 0-10 amino acid mutations. In some embodiments, the amino acid mutation in a FR region variant of light chain variable region is one or more selected from the group consisting of F71Y, K49Y, Y36F, and L47W. In some embodiments, the antibody light chain is selected from SEQ ID NO: 9 and a variant thereof. Furthermore, the IL-17A binding agent comprises light chain constant region derived from human κ, λ chain or a variant thereof.

According to some embodiments of the present invention, the IL-17A binding agent, wherein the antibody heavy chain variable region thereof further comprises the heavy chain FR region derived from human IgG1, IgG2, IgG3, IgG4 or a variant thereof; In some embodiments, the heavy chain FR region of the antibody heavy chain variable region is the FR region of human germline heavy chain VH1-18, whose amino acid sequence is shown in SEQ ID NO: 3, or a variant thereof; In some embodiments, a FR region variant of antibody heavy chain variable region refers to human germline heavy chain VH1-18 with 0-10 amino acid mutations; In some embodiments, the amino acid mutation in FR region variants of heavy chain variable region is one or more selected from the group consisting of: A93T, T71A, M48I, V67A, M69L, T73D, and S76N; In some embodiments, the antibody heavy chain is selected from SEQ ID NO: 5, SEQ ID NO: 6, SEQ ID NO: 7 and SEQ ID NO: 8. Furthermore, the IL-17A binding agent comprises heavy chain constant region derived from human IgG1, IgG2, IgG3, IgG4 or a variant thereof.

Furthermore, according to some embodiments of the present invention, provided is a vector expressing IL-17A mentioned above. The host cells express and secrete the IL-17A binding agent after being transfected with the vector.

According to some embodiments of the present invention, the vector comprises nucleotide encoding the IL-17A binding agent of the present invention.

Furthermore, according to some embodiments of the present invention, provided is a pharmaceutical composition, which comprises the IL-17A binding agent as described above and a pharmaceutically acceptable excipient, diluent or carrier.

Furthermore, according to some embodiments, the present invention also provides use of the above IL-17A binding agent, or pharmaceutical composition containing the same, in the preparation of a medicament for treatment of IL-17 mediated diseases or disorders. The diseases are inflammatory or autoimmune diseases; the diseases are selected from psoriasis, psoriatic arthritis, ankylosing spondylitis, multiple sclerosis, inflammatory arthritis; the inflammatory disease is preferably inflammatory arthritis. The inflammatory arthritis is selected from osteoarthritis, rheumatoid arthritis, rheumatic arthritis or osteoporosis, preferably rheumatic arthritis.

According to some embodiments, the present invention also provides use of the above IL-17A antibody, or pharmaceutical composition containing the same, in the preparation of a medicament for treatment of IL-17 mediated diseases or disorders. The diseases are inflammatory or autoimmune diseases. The inflammatory disease is preferably inflammatory arthritis. The inflammatory arthritis is selected from osteoarthritis, rheumatoid arthritis or osteoporosis.

According to some embodiments, the present invention also provides a method for treating a disease or disorder mediated by IL-17, comprising administering to a subject in need thereof a therapeutically effective amount of IL-17A binding agent as described above or humanized IL-17A antibody or pharmaceutical composition containing the same.

So that the invention may be more readily understood, certain technical and scientific terms are specifically defined below. Unless specifically defined elsewhere in this document, all other technical and scientific terms used herein have the meaning commonly understood by one of ordinary skill in the art to which this invention belongs.

### I. TERMS

As used herein, the single-letter code and the three-letter code for amino acids are as described in J. Biol. Chem, 243, (1968) p3558.

As used herein, "binding agent" refers to soluble receptor or fragments or analogs thereof, or antibodies or fragments thereof or analogs thereof capable of binding to the target. "IL-17A binding agent" according to the present invention, refers to antibody or fragment or analog thereof capable of specifically recognizing IL-17A and binding to IL-17A.

The term "IL-17A" generally refers to natural or recombinant human IL-17A, and non-human homologues of human IL-17A.Unless otherwise indicated, the molecular weight of IL-17A homodimer is adopted (for example, 30KDa for human IL-17A) for calculating the molar concentration of IL-17A.

As used herein, "Antibody" refers to immunoglobulin, a four-peptide chain structure consisting of two identical heavy chains and two identical light chains connected via disulfide bond. Immunoglobulin heavy chain constant regions exhibit different amino acid components and orders, hence present different antigenicity. Accordingly, immunoglobulins can be divided into five categories, or called immunoglobulin isotypes, namely IgM, IgD, IgG, IgA and IgE. According to its amino acid components of hinge region and the number and location of heavy chain disulfide bonds, Ig in the same category can further be divided into different sub-types, for example, IgG can be divided into IgG1, IgG2, IgG3, and IgG4. Light chain can be divided into κ or λ chain by different constant regions.

The region of about 110 amino acid sequences near the N-terminus of the antibody heavy and light chains, changes largely, known as variable region (V region); the region of the rest amino acid sequence near the C-terminus is relative stable, known as constant region (C region).Variable region comprises three hypervariable regions (HVR) and four relatively conserved FR regions (FR). Three hypervariable regions determine the specificity of the antibody, also known as complementarity determining region (CDR).Each light chain variable region (LCVR) and each heavy chain variable region (HCVR) is composed of three CDR regions and four FR regions, sequential order from the amino terminus to the carboxyl terminus is: FR1, CDR1, FR2, CDR2, FR3, CDR3, and FR4. Three light chain CDR regions, namely light chain hypervariable regions (LCDR), refer to LCDR1, LCDR2, and LCDR3; three heavy chain CDR regions, namely heavy chain hypervariable regions (LCDR), refer to HCDR1, HCDR2 and HCDR3. The number and location of CDR region amino acid residues in LCVR and HCVR regions of the antibody or antigen binding fragment herein comply with known Kabat numbering criteria (LCDR1-3, HCDE2-3), or comply with kabat and chothia numbering criteria (HCDR1).

As used herein, "antigen-binding fragment" refers to a Fab fragment, Fab' fragment, F(ab')₂ fragment or a single Fv fragment having antigen-binding activity. Fv antibody is a minimum antibody fragment comprising a heavy chain variable region, a light chain variable region and all antigen-binding sites, without constant region. Generally, Fv antibody further comprises a polypeptide linker between the VH and VL domains, and is capable of forming a structure required for antigen binding.

As used herein, the term "antigen determinant" of the present invention, refers to the three-dimensional sites, which are discrete on the antigen, and recognized by the antibody or antigen binding fragment of the present invention.

"Administration" and "treatment," as they apply to animals, human, experimental subjects, cells, tissues, organs, or biological fluid, refer to contact animals, human, subjects, cells, tissues, organs, or biological fluid with an exogenous medicaments, therapeutic agents, diagnostic agents, or compositions. "Administration" and "treatment" can refer to, e.g., therapeutic, pharmacokinetic, diagnostic, research, and experimental methods. Treatment of cells encompasses contacting cells with an agent, as well as contacting fluid with an agent, where the fluid is in contact with the cells. "Administration" and "treatment" also mean in vitro and ex vivo treatment of, e.g., cells, by an agent, a diagnostic or binding composition, or by another cells. "Treatment," as it applies to human, veterinary, or research subjects, refers to therapeutic treatment, prophylactic or preventative measures, for research and diagnostic applications. "Treatment" as it applies to human, veterinary, or research subjects, or cells, tissues, or organs, encompasses contacting human or animal subjects, cells, tissues, physiological compartments, or physiological fluid with an IL- 17A agonist or an IL-17 A antagonist. "Treatment of cells" also encompasses situations where the IL-17A agonist or IL-17A antagonist is contacted with IL- 17A receptor, e.g., in the fluid phase or colloidal phase, and also encompasses situations where the agonist or antagonist is not contacted with the cells or the receptors.

"Treat" means to administer a therapeutic agent, such as a composition containing any of the binding compounds of the present invention, internally or externally to a patient having one or more disease symptoms for which the agent has known therapeutic activity. Typically, the agent is administered in an amount effective to alleviate one or more disease symptoms in the patient or population to be treated, either by inducing the regression of or inhibiting the progression of such symptom(s) by any clinically measurable degree. The amount of a therapeutic agent that is effective to alleviate any particular disease symptom (also referred to as the "therapeutically effective amount") may vary according to various factors, such as the disease state, age, and weight of the patient, and the ability of the drug to elicit a desired response in the patient.

Four variants of human IL-17 A protein are mentioned herein:
1) As used herein, the terms "human IL-17A (huIL-17A)" and "natural human IL-17A" refer to the mature forms (i.e. residues 24-155) of human IL-17A protein with accession numbers NP_002181 and AAT22064, and naturally occurring variants and polymorphisms thereof.
2) As used herein, the term "rhIL-17A" refers to a recombinant human IL-17A, this nomenclature is adopted for convenience in referring to various forms of IL-17 A, and may not match usage in the literature.
3) As used herein, the term "His-huIL-17A" refers to a recombinant human IL-17A having an N-terminal His tag appended, "FLAG-huIL-17A" refers to a recombinant human IL-17A having an N-terminal FLAG tag appended. In some experiments the FLAG-huIL-17A is biotinylated.
4) R&D Systems human IL-17A mentioned herein is a recombinant human IL-17A purchased from R&D Systerms.

As used herein, the term "monoclonal antibody" refers to an antibody secreted by a clone derived from a single cell. Monoclonal antibodies are highly specific and are directed against a single epitope. The cell is not limited to eukaryotic, prokaryotic, or phage clonal cell lines.

The monoclonal antibody herein specifically includes "chimeric" antibody, in which a portion of the heavy and/or light chain is identical with or homologous to the corresponding sequences of antibodies derived from a particular species or belonging to a particular antibody type or subtype, while the remainder of the chain(s) is identical with or homologous to the corresponding sequences of antibodies derived from another species or belonging to another antibody type or subtype, as well as fragment of such antibody, as long as they exhibit the desired biological activity.

As used herein, the term "humanized antibody" is a variable region-modified form of the murine antibody according to the present invention, having CDR regions derived from (or substantially derived from) a non-human antibody (preferably a mouse monoclonal antibody), and FR regions and constant regions substantially derived from human antibody; that is, CDR region sequences of murine antibody are grafted onto different types of human germline antibody framework sequences. Such framework sequences can be obtained from public DNA databases or published references which include germline antibody gene sequences. For example, germline DNA sequences of human heavy variable region genes and light chain variable region genes can be found in the human germline sequence database "VBase" (available on the Internet www.mrccpe.com.ac.uk/vbase), as well as found in Kabat, EA, etc. 1991 Sequences of Proteins of Immunological Interest, 5th Ed. Because CDR sequences are responsible for most antibody-antigen interactions, it is feasible to construct an expression vector to express recombinant antibody which can mimic specific feature of a naturally occurring antibody.

"Optional" or "optionally" means that the following event or situation may but not necessarily occur, and the description includes the instances in which the event or situation does or does not occur. For example, "optionally contains 1-3 antibody heavy chain variable regions" means the antibody heavy chain variable region with specific sequences may be, but not necessarily be, present, if present, there may be 1, 2 or 3.

Transformation of the host cell with the recombinant DNA may be carried out by conventional techniques well known to those skilled in the art. The obtained transformants are cultured by using conventional methods to express the polypeptide encoded by the gene of the invention. Culture medium may be selected from various conventional culture mediums based on the host cells used. The host cells grow under proper conditions.

### II. Antibodies Specific for Human IL-17A

The present invention provides engineered anti-IL-17A antibodies and uses thereof to treat various inflammatory, immune and proliferative disorders, including rheumatoid arthritis (RA), osteoarthritis, rheumatoid arthritis osteoporosis, inflammatory fibrosis (e.g., scleroderma, lung fibrosis, and cirrhosis), inflammatory bowel disorders (e.g., Crohn's disease, ulcerative colitis and inflammatory bowel disease), asthma (including allergic asthma), allergies, COPD, multiple sclerosis, psoriasis and cancer.

Any suitable method for generating monoclonal antibodies may be used to generate the anti-IL-17A antibodies of the present invention. For example, an animal recipient may be immunized with a linked or e.g. naturally occurring IL-17A homodimer, or a fragment thereof. Any suitable method for immunization can be used. Such methods may include adjuvants, other immunostimulants, repeated booster immunizations, and the use of one or more immunization routes.

Any suitable form of IL-17A can be used as the immunogen (antigen) for the generation of the non-human antibody specific for IL-17A, the antibody can be screened for its biological activity. The eliciting immunogen may be full-length mature human IL-17A, including linked naturally occurring homodimers, or peptides thereof encompassing single epitope or multiple epitopes. The immunogen may be used alone or in combination with one or more immunogenicity enhancing agents known in the art. The immunogen may be purified from a natural source or produced in genetically modified cells. DNA encoding the immunogen may be derived from genomic or non-genomic (e.g., cDNA) DNAs. Suitable genetic vectors may be used to express the DNAs encoding the immunogen, said vectors include but not limited to adenoviral vectors, adenoassociated viral vectors, baculoviral vectors, plasmids, and non-viral vectors.

An exemplary method of producing anti-human IL-17A antibodies of the present invention is described at Example 1.

### III. Humanization of IL-17A Specific Antibodies

The humanized antibody can be selected from any type of immunoglobulins, including IgM, IgG, IgD, IgA, and IgE. In one embodiment, the antibody is an IgG antibody. Any isotype of IgG can be used, including IgG1, IgG2, IgG3, and IgG4. Variants of the IgG isotypes are also contemplated. The humanized antibody may comprise sequences derived from more than one type or isotype. Optimization of the necessary constant domain sequences to generate the desired biologic activity is readily achieved by screening the antibodies in the biological assays described below in the Examples.

Likewise, any type of light chain can be used in the compounds and methods herein. Specifically, kappa (κ), lambda (λ), or a variant thereof is useful in the present compounds and methods.

An exemplary method of humanizing anti-human IL-17A antibodies of the present invention is described at Example 2.

### DETAILED DESCRIPTION OF THE INVENTION

Hereinafter, the present invention is further described with reference to examples; however, the scope of the present invention is not limited thereto.

In the examples of the present invention, where specific conditions are not described, the experiments are generally conducted under conventional conditions, or under conditions proposed by the material or product manufacturers. See Sambrook et al., Molecular Cloning, A Laboratory Manual, Cold Spring Harbor Laboratory; Current Protocols in Molecular Biology, Ausubel et al, Greene Publishing Associates, Wiley Interscience, NY. Where the source of the reagents is not specifically given, the reagents are commercially available conventional reagents.

### Example 1 mouse anti-human IL-17A monoclonal antibody

Monoclonal antibodies against human IL-17A were obtained as follows. 6-8 weeks old female BALB/c mice (Shanghai Super B&K Laboratory Animal Corp. Ltd, laboratory animal production Certificate No: SCXK (HU) 2008-0016) and 6-8 week old female SJL mice (Beijing Weitong Lihua Experimental Animal Technology Co. Ltd, laboratory animal production Certificate No: SCXK (Beijing) 2012-0001) were divided into two groups, high dose group and low dose group. 10 BALB/c mice and 10 SJL mice were for each group.

The high and low dose group were serially immunized with natural hIL-17A variant (His-hIL-17A, the amino acid sequence of hIL -17A refers to human IL-17A protein Genbank accession number NP-002181, the resulting protein was purified by Ni affinity column (Superdex) 75SEC sequentially), which were additionally His-tagged in the N-terminus and generated by HEK293E (293-EBNA, Invitrogen, Lot Num: 493985) expression system. The inoculations were performed on day 0, 14, 35, and 56.

On day 0, high dose group were administered with His-huIL-17A, 500µg/mouse, via subcutaneous (s.c.) injection, and administered with Complete Freund's Adjuvant (CFA) via intraperitoneal (i.p.) injection at the same time. On day 14 and 35, 25 µg/mouse His-hIL-17A was administered via s.c. injections, at the same time, Incomplete Freund's Adjuvant (IFA) was administrated via i.p. injection . On day 56, before fusing the splenocytes, a booster immunization was performed by i.p. injection with 25 µg/mouse His-hIL-17A dissolved in saline. The time schedule and method for the immunization of low dose group are the same as those for high dose group, except that administered dose of His-hIL-17A on day 0 was 10µg/mouse, administered dose of His-hIL-17A on day 14, 35, and 56 was 5µg/mouse.

Blood tests were performed on day 22 and day 43. Mice serum was tested by ELISA Test described in Example 1 to determine the antibody titers in serum. On day 56, mice with higher antibody titers in serum were selected for splenocyte fusion. Hybridoma was obtained by fusing splenic lymphocyte with myeloma cells Sp2/0 cells (ATCC® CRL-8287™) by using optimized PEG-mediated fusion procedure.

The procedures for immunization were as follows:
Scheme 1, high dose, 10 Balb/c mice and 10 SJL mice, the procedures were as follows:

| Day 0 | Pre-blood sampling 15-30µL serum/mouse; primary immunization, IP, CFA 50µg/mouse |
|---|---|
| 14 | Boost 1 (booster immunization 1): IP, IFA 25µg/mouse |
| 21 | Blood sampling (15-30µL serum/mouse) |
| 22 | ELISA test |
| 35 | Boost 2 (booster immunization 2): IP, IFA 25µg/mouse |
| 42 | Blood sampling (15-30µL serum/mouse) |
| 43 | ELISA test |
| 44 | Data analysis and interim conclusion |
| 56 | Pre-fusion booster immunization, IP, 25µg/mouse of saline |

Scheme 2, low dose, the procedures were as follows:

| Day 0 | Pre-blood sampling 15-30µL serum/mouse; primary immunization, IP, CFA 10µg/mouse |
|---|---|
| 14 | Boost 1 (booster immunization 1): IP, IFA 5µg/mouse |
| 21 | Blood sampling (15-30µL serum/mouse) |
| 22 | ELISA test |
| 35 | Boost 2 (booster immunization 2): IP, IFA 5µg/mouse |
| 42 | Blood sampling (15-30µL serum/mouse) |
| 43 | ELISA test |
| 44 | Data analysis and interim conclusion |
| 56 | Pre-fusion booster immunization, IP, 5µg/mouse of saline |

Primary screening of the resulting hybridomas was performed by antigen-antibody indirect ELISA test in Test Example 1. Monoclonal cell strains were obtained via limiting dilution of positive cell strains.

The obtained monoclonal cell lines were further screened, including:
1. Receptor blocking test, see Test Example 2, the results were shown in Table 5, monoclonal cell line IL17-mAb049 having activity superior to the positive control was screened and obtained;
2. Affinity test: see Test Example 3, the results were shown in Table 6, which revealed that monoclonal cell line IL17-mAb049 screened and obtained in the present invention showed comparable or better activity when compared to the positive control;
3. Bioassay at cellular level (GROα analysis): see Test Example 4, the results were shown in Table 8, which revealed that monoclonal cell line IL17-mAb049 screened and obtained in the present invention showed comparable or better activity when compared to the positive control.

Twelve the monoclones were studied further after the first and second screens. One lead monoclone (lead mAb) of IL17-mAb049 was selected by epitope grouping, biological activity test. The specific sequences of heavy chain (VH) and light chain (LH) of murine IL-17A mouse antibody mAb049 (IL-17mAb) were as follows:

IL-17 mAb049 VH SEQ ID NO: 1
HVQLQQSGADLVRPGASVTLSCKASGYIFTDYEVHWVKQTPVHGLEWIGVIDPGTG
GVAYNQKFEGKATLTADDSSNTAYMELRSLTSEDSAVYYCTRYSLFYGSSPYAMDY
WGQGTSVTVSS

IL-17mAb049 VL SEQ ID NO: 2
QIVLTQSPAIMSASPGEKVTITCSASSSVNYMHWFQQKPGTSPKLWIYRTSNLASGVP
VRFSGSGSGTSYSLTISRMEAEDAATYYCQQRSSYPWTFGGGTNLEIK

### Example 2 Humanization of Murine-Anti-Human IL-17A Antibodies

The humanization of murine-anti-human IL-17A monoclonal antibody mAb049 was performed essentially as described in a lot of literatures known to the public in the art. Briefly, human constant domains were used to replace the parental (murine antibody) constant domains. The human germline sequences used for humanization were selected according to homology between the murine antibody and human antibody.

### 1. CDR regions of murine anti-IL-17A antibody

VH/VL CDR amino acid residues were identified and annotated by the Kabat numbering system. CDR sequences of murine mAb049 in the present invention were listed in the following table:

**Table 1: CDR sequences of mouse anti-IL-17A antibody**

| Domain | | mAb049 | |
|---|---|---|---|
| | | Sequence | SEQ ID NO |
| VH | CDR1 | DYEVH | 10 |
| | CDR2 | VIDPGTGGVAYNQKFEG | 11 |
| | CDR3 | YSLFYGSSPYAMDY | 12 |
| VL | CDR1 | SASSSVNYMH | 13 |
| | CDR2 | RTSNLAS | 14 |
| | CDR3 | QQRSSYPWT | 15 |

### 2. Selection of Human germline FR sequences

On the basis of typical structures of the obtained murine antibody VH/VL CDRs, the sequences of heavy and light chain variable regions were compared with antibody database. Human germline heavy chain VH1-18 (SEQ ID NO: 3) and light chain A10 (SEQ ID NO: 4) with high homology were obtained and used as humanized FR sequences. Specific sequences are as follows:

VH1-18 SEQ ID NO: 3
QVQLVQSGAEVKKPGASVKVSCKASGYTFTSYGISWVRQAPGQGLEWMGWISAYN
GNTNYAQKLQGRVTMTTDTSTSTAYMELRSLRSDDTAVYYCAR

A10 SEQ ID NO: 4
EIVLTQSPDFQSVTPKEKVTITCRASQSIGSSLHWYQQKPDQSPKLLIKYASQSFSGVP
SRFSGSGSGTDFTLTINSLEAEDAATYYCHQSSSLP

### 3. Design of humanized antibodies:

The amino acid residues forming the ring conformation and VH interface were determined. Taking the following factors into consideration, Q1E mutation was to eliminate the N-terminal pyroglutamic acid formation. Mutations also include those maintaining consistency within the selected VH family, in order to maintain CDR typical structure and VH/VL interface, and avoid N-glycosylated pattern (N-{P}-S/ T) present in the humanized structure.

Design of Humanized mutations in variable regions of murine antibody mAb049 was summarized as follows:

**Table 2: Humanized sites designed in murine antibody mAb049**

| Humanized sites designed in heavy chain | | Humanized sites designed in light chain | |
|---|---|---|---|
| VH (VH1-18) + JH4/FW4 | | Vk(A10) + JK2/FW4 | |
| Mutation type | Humanized back mutation site | Mutation type | Humanized back mutation site |
| Hu049 VH.1 | CDR-grafted* | Hu049 Vk.1 | CDR-grafted* |
| Hu049 VH.1A | A93T | Hu049 Vk.1A | F71Y |
| Hu049 VH.1B | A93T, T71A | Hu049 Vk.1B | F71Y, K49Y |
| Hu049 VH.1C | A93T, T71A, M48I | Hu049 Vk.1C | F71Y, K49Y, Y36F, L47W |
| Hu049 VH.1D | A93T, T71A M48I, V67A, M69L, T73D, S76N | | |

| | | | |
|---|---|---|---|
| NOTE: For example, A93T denotes back mutation from 93A to T according to Kabat numbering system. * Indicating that the murine antibody CDR was implanted into human germline FR sequences. | | | |

**Table 3: Murine antibody mAb049 humanized sequences**

| | Hu049 VH.1 | Hu049 VH.1A | Hu049 VH.1B | Hu049 VH.1C | Hu049 VH.1D |
|---|---|---|---|---|---|
| Hu049 VK.1 | Hu049-1 | Hu049-2 | Hu049-3 | Hu049-4 | Hu049-5 |
| Hu049 VK.1A | Hu049-6 | Hu049-7 | Hu049-8 | Hu049-9 | Hu049-10 |
| Hu049 VK.1B | Hu049-11 | Hu049-12 | Hu049-13 | Hu049-14 | Hu049-15 |
| Hu049 VK.1C | Hu049-16 | Hu049-17 | Hu049-18 | Hu049-19 | Hu049-20 |

| | | | | | |
|---|---|---|---|---|---|
| NOTE: This table shows various sequence combinations of different mutations. For example, Hu049-8 indicates two mutations (Hu049VK.1A and Hu049VH.1B) are present in humanized murine antibody mAb049, and so on. | | | | | |

### 4. Expression and purification of humanized antibody

The above-mentioned antibodies were cloned, expressed and purified by genetically recombinant methods. Humanized antibodies with good performance were eventually selected by ELISA, receptor binding inhibition assay, Biacore, cell viability test etc. Specific antibodies are indicated in the following table:

**Table 4: components of humanized IL-17A antibody**

| Antibody | Heavy chain | SEQ ID NO | Light chain | SEQ ID NO |
|---|---|---|---|---|
| Hu049-17 | Hu049-17.VH | SEQ ID NO: 5 | Hu049 VL | SEQ ID NO: 9 |
| Hu049-18 | Hu049-18.VH | SEQ ID NO: 6 | | |
| Hu049-19 | Hu049-19.VH | SEQ ID NO: 7 | | |
| Hu049-20 | Hu049-20.VH | SEQ ID NO: 8 | | |

Specific sequences of humanized antibody mAb049are listed below:

Hu049-17.VH SEQ ID NO: 5
EVQLVQSGAEVKKPGASVKVSCKASGYTFTDYEVHWVRQAPGQGLEWMGVIDPG
TGGVAYNQKFEGRVTMTTDTSTSTAYMELRSLRSDDTAVYYCTRYS
LFYGSSPYAMDYWGQGTLVTVSS

Hu049-18.VH SEQ ID NO: 6
EVQLVQSGAEVKKPGASVKVSCKASGYTFTDYEVHWVRQAPGQGLEWMGVIDPG
TGGVAYNQKFEGRVTMTADTSTSTAYMELRSLRSDDTAVYYCTRYS
LFYGSSPYAMDYWGQGTLVTVSS

Hu049-19.VH SEQ ID NO: 7
EVQLVQSGAEVKKPGASVKVSCKASGYTFTDYEVHWVRQAPGQGLEWIGVIDPGT
GGVAYNQKFEGRVTMTADTSTSTAYMELRSLRSDDTAVYYCTRYSLFYGSSPYAM
DYWGQGTLVTVSS

Hu049-20.VH SEQ ID NO: 8
EVQLVQSGAEVKKPGASVKVSCKASGYTFTDYEVHWVRQAPGQGLEWIGVIDPGT
GGVAYNQKFEGRATLTADDSTNTAYMELRSLRSDDTAVYYCTRYS
LFYGSSPYAMDYWGQGTLVTVSS

Hu049VL SEQ ID NO: 9
EIVLTQSPDFQSVTPKEKVTITCSASSSVNYMHWFQQKPDQSPKLWIYRTSNLASGV
PSRFSGSGSGTDYTLTINSLEAEDAATYYCQQRSSYPWTFGQGTKLEIKR

### Example 3 In vivo pharmacokinetics and pharmacodynamics test of humanized anti-IL-17 antibody

Human IL-17 can bind to and stimulate mouse IL-17 receptor, leading to increase and subsequent secretion of chemokines in male mice KC (CXCL1). Experiments with various time and various doses were performed to identify optimal dose of human IL-17 and the best time for inducing mice KC (see Test Example 5). These experiments show that 150mg/kg of human IL-17 and 2 hours after IL-17 administration induces the highest level of KC in mouse serum. Full-length antibodies of the present invention were intravenously administered to mice at the concentration of 3, 30, 300, 3000 µg/kg, 20 hours before the subcutaneous injection of human IL-17. 2 hours after human IL-17 administration, the mice were sacrificed and KC level was determined by using a commercially available kit, by ELASA according to the manufacturer's specification (Mouse CXCL1 / KC Quantikine ELISA Kit, R & D SYSTEM, # SMKC00B). Isotype-matched antibody was used as negative control. Antibodies block the ability of human IL-17 to stimulate mouse IL-17 receptor, resulting in the inhibition of increased KC in a dose-dependent manner in mice. Compared to the ineffective control antibody, the antibody Hu049-18 of the present invention reduced the average KC level to about 1/6 under the described conditions at the dose of 3000µg/mice.

Serum pharmacokinetics in rats and rhesus monkeys was determined after intravenous or subcutaneous administration of the antibody Hu049-18 of the present invention (see Test Example 6). In rats, the half-life was 9.91 days after intravenous administration of 5mg/kg, and the half-life was 11.5 days after subcutaneous administration of 5mg/kg. In macaque, the half-life was 24.4 days after intravenous administration of 1mg/kg.

### Test Examples

### Test Example 1 Indirect ELSIA

### Purpose:

Indirect ELISA method was selected for ensuring the selection of antibodies which can recognize conformational epitope, and for screening the mouse hybridomas from Example 1 of the present invention.

### Materials:

Human IL-17A (hIL-17A) was cloned according to methods known in the art, by utilizing the human IL-17A protein sequences with Genbank Accession No. NP-002181, and transiently transfected into HEK293E cells for expression.

Human IL-17A/F (heterodimer, hIL-17A/F) was cloned according to methods known in the art, by utilizing the human IL-17A protein sequences with Genbank Accession No. NP-002181 and human IL-17F protein sequences with Genbank Accession No. NP_443104, and transiently transfected into HEK293E cells for expression.

As the positive controls, Lilly and Novartis murine anti-IL-17 antibodies (Lilly mAb, Novartis mAb) were cloned by the murine sequences disclosed in US7,838,638B2 (LY 2439821) and US 7,807,155B2 (AIN 457), respectively, and transiently transfected into HEK293E cells for expression.

Murine antibodies mAbs derived from mouse hybridoma disclosed in Example 1of the present invention.

### Protocol:

1. Microtitration plates were directly coated with 1µg/ml of streptavidin, at 4 °C overnight;
2. Microtitration plate were blocked with 300µl of PBST containing 2% BSA (v/v), thermostatically incubated at 37°C for 1h, while the uncoated wells were blocked as control;
3. Washing with PBST for three times, all the washing operations were performed on Biotek (Elx 405) automatic washer;
4. 100µl of PBS containing hIL-17A or hIL-17A/F (1µg/ ml) was added to each well, thermostatically incubated at 37 °C for 1h;
5. PBST washing for 3 times.
6. Positive controls Lilly mAb and Novartis mAb or murine antibody mAbs of the present invention were titrated under 1: 5 dilution, the initial concentration is 1µg / ml. 100µl of diluted positive control or murine antibody of the present invention was added to each well, thermostatically incubated at 37 °C for 1h. Each concentration was titrated in duplicate well;
7. PBST washing for 3 times;
8. 100µl of HRP anti-murine secondary antibody (Santa Cruz Cat.No.sc-2005) (1: 5000) was added to each well, thermostatically incubated at 37 °C for 1h;
9. PBST washing for 3 times. 100µl of TMB Substrate was added to each well, thermostatically incubated at 37 °C for 5min. Then the reaction was stopped by addition of 100µl 2M H₂SO₄ each well;
10. The OD value at 450nm wavelength was read on ELISA microplate reader (Molecular Devices, Spectra Max).
11. The OD values of murine antibody mAbs were compared with those of the positive controls. Monoclonal cell lines with a ratio greater than 1 were screened, wherein IL17-mAb049 was included.

### Test Example 2 IL-17 receptor blocking assay (RBA)

### Purpose:

The purpose of receptor blocking assay is to select the antibodies capable of blocking the binding of IL-17 to IL-17 receptor (e.g., hIL-17RA). The test is based on functional test, and it can be used for hybridoma high-throughput screening.

### Materials and equipments:

Anti-human Fc antibody (goat anti-human IgG-Fc fragment specific antibody (available from Jackson Immunoresearch, 109-005-008))

Human IL-17RA-Fc used herein is cloned according to methods known in the art, by utilizing the human IL-17A receptor amino acid sequences with Genbank ID No. ADY18334.1, and transiently transfected into HEK293E cells for expression, wherein the Fc fragments were obtained from human IgG1.

As the positive controls, Lilly and Novartis murine anti-IL-17 antibodies (Lilly mAb, Novartis mAb) were cloned according to the murine sequences disclosed in US 7,838,638B2 (LY 2439821) and US 7,807,155B2 (AIN 457), and transiently transfected into HEK293E cells for expression.
mIgG: Murine IgG (Millipore Cat.No.PP54), used as blank control
ELISA plate reader: Molecular Devices, Spectra Max

Murine monoclonal cell strains obtained from Example 1 of the present invention .

### Protocol:

1. Microtitration plates were directly coated with 10µg/ml of Anti-human Fc antibody, incubated at 4°C overnight;
2. Microtitration plates were blocked with 300µl of PBST containing 2% BSA (v/v), thermostatically incubated at 37 °C for 1h, while the uncoated well was blocked as control;
3. Washing with PBST for three times, all the washing operations were performed on Biotek (Elx 405) automatic washer;
4. 100µl of PBS containing IL-17 RA-Fc (60ng/ml) was added to each well, thermostatically incubated at 37 °C for 2h;
5. PBST washing for 3 times.
6. Positive controls Lilly mAb and Novartis mAb or antibodies of the present invention were diluted at the ratio of 1: 5, the initial concentration was 40 µg/ml. mIgG was diluted with the same method. 50µl of diluted positive control or murine antibody of the present invention or mIgG was added to each well, meanwhile, 50µl of 0.2nM biotin-labeled IL-17A was added to the diluted positive control or the antibody of the present invention, mixed gently and thermostatically incubated at 37 °C for 1h.
7. PBST washing for 3 times;
8. 100µl of HRP-labeled streptavidin complex (1:5000) was added to each well, thermostatically incubated at 37 °C for 1h;
9. PBST washing for 3 times. 100µl of TMB Substrate was added to each well, thermostatically incubated at 37 °C for 5min. Then the reaction was stopped with addition of 100µl 2M H₂SO₄ each well;
10. The OD value at 450nm wavelength was read on ELISA microplate reader.
11. IC₅₀ value of the antibody to be tested was calculated for blocking the binding of IL-17 to IL-17 receptor.

IC₅₀ value (the antibody concentration when OD value reduced to 50%, i.e. RBA) was obtained according to the gradient curve of OD values versus antibody concentration.

### Experimental Results:

According to the above method, the hybridoma obtained in Example 1 was screened to obtain a murine monoclonal antibody, designated as IL17-mAb049, the results are as follows:

**Table 5:**

| Antibody | huIL-17 RBA (nM) |
|---|---|
| Lilly mAb | 0.17 |
| Novartis mAb | 1.56 |
| IL17-mAb049 | 0.07 |

Conclusion: The murine antibody IL17-mAb 049 screened from hybridomas showed better activity than positive antibodies Lilly mAb and Novartis mAb.

### Test Example 3 Affinity Test

### Purpose:

The BIACORE method was used in the experiment for determining antigen-antibody binding kinetics and affinity.

### Materials and equipments:

### 1.1 Proteins:

Human IL-17A (hIL-17A) was cloned according to methods known in the art, by utilizing the human IL-17A protein sequences with Genbank Accession No. NP-002181, and transiently transfected into HEK293E cells for expression.

Human IL-17A/F (heterodimer, hIL-17A/F) was cloned according to methods known in the art, by utilizing the human IL-17A protein sequences with Genbank Accession No. NP-002181 and human IL-17F protein sequences with Genbank Accession No. NP_443104, and transiently transfected into HEK293E cells for expression.

Mouse IL-17A (Mu IL-17A) and rat IL-17A (Rat IL-17A) were cloned according to methods known in the art, by utilizing the mouse IL-17A protein with Genbank Accession No. NP_034682 and rat IL-17A protein with Genbank Accession No. NP_001100367, respectively, and transiently transfected into HEK293E cells for expression.

As positive controls, Lilly and Novartis murine anti-IL-17 antibodies (Lilly mAb, Novartis mAb) were cloned according to the murine sequences disclosed in US 7,838,638B2 (LY 2439821) and US 7,807,155B2 (AIN 457), respectively, and transiently transfected into HEK293E cells for expression.

As a positive control, Lilly humanized anti-IL-17 antibody (Lilly mAb(hu),) was cloned according to the humanized sequences disclosed in US 7,838,638B2 (LY 2439821), and transiently transfected into HEK293E cells for expression.

Murine monoclonal cell strains obtained from Example 1 of the present invention.

Humanized IL-17 antibodies obtained from Example 2 of the present invention.
1.2BIACORE Model: BIACORE X 100, GE;
1.3BIACORE chips and reagents (Trade names were listed hereinafter, no acknowledged translation):

| Materials and Reagents | Company | Product list |
|---|---|---|
| 1. Sensor Chip CM5 Research Grade | GE Healthcare | BR-1000-14 |
| 2. Amine Coupling Kit | GE Healthcare | BR-1000-50 |
| 3. HBS buffer BIA Certified | GE Healthcare | BR-1001-88 |
| 4. Acetate (100ml) | GE Healthcare | BR-1003-51 |
| 5. Mouse Antibody Capture Kit | GE Healthcare | BR-1008-38 |
| 6. Regeneration buffer Glycine 1.5 | GE Healthcare | BR-1003-54 |
| 7. BIAmaintenance Kit | GE Healthcare | BR-1006-66 |

### Protocol:

1. The antibody of the present invention was immobilized on CM5 chip: 1:1 50mM NHS: 200mM EDC was prepared and injected into FC2 (Flow cell 2) channel at a rate of 10µL/min, for 7min, to activate CM5 sensor chip. The Antibody of the present invention was dissolved in 10mM sodium acetate buffer at a concentration of 30µg/ml, PH 5.0, and injected into activated chip (HBS-EP mobile phase buffer: 10 mM HEPES, 150 mM NaCl, 3.4 mM EDTA, 0.005% surfactant P20, pH 7.4) at a rate of 5µL/min. 1M ethanolamine was injected at a rate of 10µL/min, for 7min, to seal the remaining activated coupling positions. About 8000RU was generated.
2. Binding kinetics Test: FC1 (Flow cell 1) was used as reference channel, FC2 (Flow cell 2) was used as sample channel, murine or humanized control antibody or the antibody of the present invention was captured at the FC2 channel in 300RU, followed by injection of different concentrations of IL-17 (including hIL-17A, MuIL-17, Rat IL-17). Cycle conditions were: injecting analytes into all FC channels at 30µl /min for 3min, dissociation for 20min, injecting 10mM Glycine, pH 1.5, for 60s (at rate of 10µl/min) for surface regeneration. The difference between signal with captured antibody and signal without captured antibody was calculated by Biacore X100 evaluation software ver 2.0 (Biacore), the running buffer was 10mM *Hepes,* 650mM NaCl, 3mM EDTA, 0.05% Tween-20.

### Experimental Results:

1. According to the above method, hybridomas obtained in Example 1 were screened, the results are as follows:

**Table 6**

| Antibody | 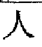 IL-17A KD (M) |
|---|---|
| Lilly mAb | 2.18E-11 |
| Novartis mAb | 4.24E-10 |
| IL17-mAb049 | 2.62E-11 |

Conclusion: The affinity of the murine antibody IL17-mAb 049 screened from hybridomas is equivalent to that of positive antibody Lilly mAb, and better than that of Novartis mAb.
2. According to the above method, humanized IL-17 antibodies obtained from Example 2 were tested, the results are as follows:

**Table 7**

| Humanized antibody | Human IL-17A KD (M) | Mu IL-17 KD (M) | Rat IL-17 KD (M) |
|---|---|---|---|
| Lilly's mAb (hu) | 1.48E-11 | | |
| Hu049-17 | <1pM | 1.37E-10 | 1.06E-09 |
| Hu049-18 | <1pM | 6.81E-11 | 4.77E-10 |
| Hu049-19 | 2.68E-12 | 7.71E-11 | 6.00E-11 |

Conclusion: The affinity of the humanized antibody was increased by 10 times than that of Lilly's positive antibody (1.48E-11M).

### Test Example 4 Bioassay at cellular level (GROα assay)

### Purpose:

The following experiment was intended to detect the cell biological activity of anti-IL-17A antibody by inhibiting IL-17-stimulated secretion of GROα from Hs27 cells with anti-IL-17A antibody.

### Materials and equipments:

Hs27 cells: ATCC Cat.No.CRL-1634 (Note: the cells cultured for more than six weeks are not recommended for bioassay);
Hs27 cell culture medium: DMEM + 10% FBS
DMEM: ATCC Cat.No.30-2002;
FBS: GIBCO Cat.No.10099, lot 8122818;
Recombinant human IL-17A (rhIL-17A): R&D Systems Cat.No.317-ILB, lot SOA161109B;
Recombinant human IL-17A/F (rhIL-17A/F): R&D System Cat No.5194-IL/CF, lot RXT101109A;
Human CXCL1/GRO alpha Quantikine PharmPak kit: R&D system Cat. No. PDGR00
Equipment: Biotek ELx808 microplate reader.

Murine monoclonal cell strain obtained from Example 1 of the present invention.

Humanized IL-17 antibody obtained from Example 2 of the present invention.

### Protocol:

### 1. Hs27 cell culture:

Hs27 cells were cultured in 50ml of DMEM +10%FBS medium in T175 flask; the cells (density of about 90%) were dilution cultured at a ratio of 1:3 every 3 days; the cells were used for bioassay within a month, or re-thawed from liquid nitrogen; the re-thawed cells should be cultured for nearly a week before bioassay.

### 2. Bioassay (IL-17A) experimental procedure

2.1 Hs27 cells were centrifuged at 950rpm for 4min (complete removal of trypsin-EDTA) and collected. Cell viability was analyzed by trypan blue stain, only cells with > 80% vitality were used for the experiment;
2.2 Medium was added into 96-well plate at 50µl/well;
2.3 Hs27 cells were diluted with DMEM+10% FBS and added into 96-well plate at a density of 10000 cells/50µl/well;
2.4 25µl of IL-17 human antibody was added into each duplicate well; the antibody was diluted at a ratio of 1:3 with the initial concentration of 10nM;
2.5 25µl of recombinant human IL-17A was added into each well with a final concentration of 0.3nM. 96-well plate was centrifuged at 500rpm for 1min;
2.6 Cells were thermostatically incubated at 37 °C for 17h;
2.7 Cell culture supernatant was collected, the concentration of GROα was detected in the supernatant by human CACL1/GRO alpha Quantikine kit (according to manufacturer's instructions);

### 3. Experimental procedures of Bioassay (IL-17A / F) :

The procedures of IL-17A/F bioassay is similar to that of IL-17A bioassay, except that IL-17A was substituted with IL-17A/F.

### Experimental Results:

1. According to the above methods, the hybridoma obtained in Example 1 was screened, the results are as follows:

**Table 8:**

| Antibody | huIL-17 Bioassay (IC50, nM) | huIL-17A/F Bioassay (IC50, nM) |
|---|---|---|
| Lilly mAb | 0.04 | 0.69 |
| Novartis mAb | 0.22 | 1.15 |
| IL17-mAb049 | 0.04 | 0.46 |

Conclusion: The biological activity of the antibody IL17-mAb049 obtained from hybridoma is equivalent to that of positive antibody Lilly mAb, and better than that of Novartis mAb.
2. According to the above methods, the humanized antibodies obtained from Example 2 were detected, the results are as follows:

**Table 9:**

| Antibody | huIL-17 Bioassay (IC50, nM) | huIL-17A/F Bioassay (IC50, nM) | Cyno IL-17A |
|---|---|---|---|
| Lilly's mAb (hu) | 0.033 | 0.83 | |
| Hu049-17 | 0.061 | 0.406 | 0.03 |
| Hu049-18 | 0.04 | 0.684 | 0.033 |
| Hu049-19 | 0.066 | 0.411 | 0.039 |
| Hu049-20 | 0.065 | 0.674 | 0.028 |

Conclusion: These results indicate that all of the humanized antibodies exhibit cell biological activity. Hu049-17, 18, 19 and 20 have IC50 (0.04nM-0.066nM) similar to that of the positive antibody (0.04nM). In addition, these antibodies display cross-reaction with cynomolgus IL-17A (IC50 is 0.03 nM-0.039 nM). The activity to human IL-17A/F is about 10 times weaker than that to IL-17A.

### Test Example 5 Neutralization test of human IL-17 in vivo

### Purpose:

The aim of neutralization test in vivo is to verify that the antibodies of the invention can block in vivo the binding of IL-17 to IL-17 receptor (e.g., hIL-17RA), thereby inhibit the CXCR1 expression induced by IL-17.

### Materials and equipments:

Protein: Human IL-17A (hIL-17A) was cloned according to methods known in the art, by utilizing the human IL-17A protein sequences with Genbank Accession No. NP-002181, and transiently transfected into HEK293E cells for expression.

As a positive control, Lilly humanized anti-IL-17 antibody (Lilly mAb (hu),) was cloned according to the humanized sequences disclosed in US 7,838,638B2 (LY 2439821), and transiently transfected into HEK293E cells for expression.
Human IgG (HuIgG): (Millipore Cat.No.AG711).
Animals: 7-week-old C57/B6 male mice (purchased from SINO-BRITSH SIPPR/BK LAB. ANIMAL LTD., CO, Certificate No.: SCXK (Shanghai) 2008-0016), 6 mice each group.
Reagents: Ab dilution solution: citrate buffer (pH 5.0): 10mM sodium citrate, 50mM NaCl
hIL-17A dilution solution: PBS (sodium phosphate buffer, pH 7.2).

Mouse CXCL1/KC Quantikine ELISA Kit, 6-well plates, R&D SYSTEM, #SMKC00B.

### Protocol:

1) Mice were divided into 15 groups, 6 each group.
2) 100uL of Hu049-18 or control antibody (HulgG or Lilly mAb (hu)) or a diluted solution was intraperitoneally (I.P.) administered to each mouse, administration doses of drug were 3000µg/kg, 300µg/kg, 30ug/kg and 3µg/kg, respectively.
3) 20 hours later, hIL-17A was subcutaneous injected (SC)at 150µg/kg , each mouse was injected with 100uL.
4) 2 hours later, blood samples were collected, placed at room temperature for 2 hours, until coagulation, or 2-8 °C overnight, until coagulation, and then centrifuged at 2000x g for 20 min. The supernatant was discarded, detection was performed immediately or aliquots of sample were stored at -20 °C. Avoid repeated freezing and thawing.
5) Samples obtained from Step 4 were measured by mouse CXCL1/KC Quantikine ELISA Kit.

### Experimental Results:

According to the above method, humanized antibody Hu049-18 obtained from Example 2 was tested, the results are as follows:

**Table 10:**

| Antibody (injection dosage 3000µg/mouse) | KC mean value(pg/mll) |
|---|---|
| HuIgG | 937 |
| Lilly mAb(hu) | 158 |
| Hu049-18 | 145 |

Conclusion: Compared to the ineffective control antibody, Hu-049-18 antibody of the present invention reduces the average KC level to about 1/6 at a dose of 3000µg/mice under the described condition. Compared with the control antibody, Hu-049-18 antibody of the present invention exhibits equivalent ability to inhibit KC at a dose of 3000µg/mice under the described condition.

### Test Example 6 Determination of the half-life (T1/2) of the antibodies in vivo

### Purpose:

To determine the pharmacokinetics parameters of the antibody Hu049-18 of the present invention in rats or cynomolgus monkeys in vivo.

### Materials and Reagents:

Protein: Human IL-17A (hIL-17A) was cloned according to methods known in the art, by utilizing the human IL-17A protein sequences with Genbank Accession No. NP-002181, and transiently transfected into HEK293E cells for expression.

As a positive control, Lilly humanized anti-IL-17 antibody (Lilly mAb (hu),) was cloned according to the humanized sequences disclosed in US 7,838,638B2 (LY 2439821), and transiently transfected into HEK293E cells for expression.
Human IgG (HuIgG): Human IgG, Polyclonal, Millipore Cat.No.AG711
Animals: 230-250g SD male rats (purchased from Shanghai SLAC laboratory Animal Co., Ltd., Certificate No: SCXK (Shanghai) 2007-0005), were divided into two groups of intravenous injection (IV) group (dorsum of foot) and subcutaneous injection (SC) group, 5 rats each group.
Macaque: 2-3kg cynomolgus monkeys (Hainan Jingang Biotechnology Co., Ltd. Certificate No: SCXK (HN) 2010-0001, 0000152.)
Reagents: antibody dilution solution: citrate buffer (pH 5.0): 10mM sodium citrate, 50mM NaCl
hIL-17A dilution solution: PBS (sodium phosphate buffer, pH 7.2)
Goat anti-human IgG (Fab-specific) peroxidase conjugated antibody, Sigma Cat.No.121M4811

### Protocol:

### 1. Procedures for detection in rat:

### (1) In vivo administration

SD rats were randomly divided into two groups (intravenous injection (IV) (dorsum of foot) group and subcutaneous injection (SC) group), 5 rats each group;
Under sterile condition, Hu049-18 was dissolved in citrate buffer solution (pH 5.0) to a final concentration of 2.5mg/mL;
Each rat was IV or SC administered with a dose of 5mg/kg;
For IV group, blood sample was taken through tail vein at 0min, 5min, 15min, 30min, 1hr, 2hr, 4hr, 8hr, 24hr, 2d, 4d, 7d, 10d, 14d, 21d, 28d after administration, 200 uL (equivalent to 80uL serum) each time; For SC group, blood sample was taken through tail vein at 0min, 30min, 1hr, 2hr, 4hr, 8hr, 12hr, 24hr, 2d, 4d, 7d, 10d, 14d, 21d, 28d after administration, 200 uL (equivalent to 80uL serum) each time;
Blood samples were collected and placed for half an hour at room temperature until coagulation, and then centrifuged at 4 °C, at 10000×g for 5 minutes. The supernatant was collected for immediate test or aliquots of sample were stored at -80°C. Avoid repeated freezing and thawing.

### (2) Serum samples obtained in step (1) were detected by ELISA

### 1) Standard curve

a) Microtitration plate was directly coated with 1µg/ml of streptavidin, at 4 °C overnight;
b) Microtitration plate was blocked with 300µl of PBST containing 2% BSA (v/v), thermostatically incubated at 37 °C for 1h, while the uncoated well was blocked as control;
c) Washing with PBST for three times, all the washing steps were performed on Biotek (Elx 405) automatic washer;
d) 100µl of PBS containing hIL-17A (0.2 µg/mL) was added to each well, thermostatically incubated at 37 °C for 1h;
e) PBST washing for 3 times.
f) Hu049-18 titration: diluted at a ratio of 1: 2 with antibody dilution, the initial concentration was 0.8µg/ml. 100µl of diluted Hu049-18 was added into each well, the standard curve was plotted. The 96-well plate was thermostatically incubated at 37 °C for 1h.
g) PBST washing for 3 times;
h) 100µl of goat anti-human IgG (Fab-specific) peroxidase conjugated antibody (Sigma Cat. No. 121M4811) (1:5000) was added to each well, thermostatically incubated at 37 °C for 1h;
i) PBST washing for 3 times. 100µl of TMB Substrate was added to each well, thermostatically incubated at 37 °C for 5min. Then the reaction was stopped with the addition of 100µl 1M HCl each well;
j) The OD value at 450nm/630nm wavelength was read on ELISA microplate reader (Molecular Devices, Spectra Max).

### 2) Sample test:

a) Microtitration plate was directly coated with 1µg/ml of streptavidin, at 4 °C overnight;
b) Microtitration plate was blocked with 300µl of PBST containing 2% BSA (v/v), thermostatically incubated at 37 °C for 1h, while the uncoated well was blocked as control;
c) Washing with PBST for three times, all the washing steps were performed on Biotek (Elx 405) automatic washer;
d) 100µl of PBS containing hIL-17A (0.2 µg/mL) was added to each well, thermostatically incubated at 37 °C for 1h;
e) PBST washing for 3 times.
f) Serum samples titration: Before the experiment, a rat serum sample was diluted in different ratios to obtain an optimal dilution ratio at which the antibody concentration in the serum was just in the middle of the standard curve. Serum samples were diluted in accordance with the optimal dilution ratio, while Hu049-18 was diluted to 25ng/mL. 100µl of diluted serum sample and Hu049-18 were added into each well, and thermostatically incubated at 37 °C for 1h. Each concentration was titrated in duplicate well;
g) PBST washing for 3 times;
h) 100µl of goat anti-human IgG (Fab-specific) peroxidase conjugated antibody (Sigma Cat. No. 121M4811) (1:5000) was added to each well, thermostatically incubated at 37 °C for 1h;
i) PBST washing for 3 times. 100µl of TMB Substrate was added to each well, thermostatically incubated at 37 °C for 5min. Then the reaction was stopped with the addition of 100µl 1M HCl each well;
j) The OD value at 450nm/630nm wavelength was read on ELISA microplate reader (Molecular Devices, Spectra Max).

### 2. Detection procedures for Macaques :

In vivo detection procedures for Macaque (Macaca fascicularis) were similar to those for rats, the differences were as follows: the administration to cynomolgus monkey was only via intravenous injection (IV) at a dose of 1mg/kg, blood sample was taken through tail vein at 0min, 5min, 15min, 30min, 1hr, 2hr, 4hr, 8hr, 24hr, 32hr, 3d, 4d, 5d, 6d, 9d, 12d, 14d, 17d, 21d, 28d, 35d after administration, 500µL each time. The serum sample after centrifugation was divided into 3 parts (ensure each of the 2 parts contains 60µL sample), and frozen at -80°C for test.

### Experimental Results:

According to the above method, the humanized antibody Hu049-18 obtained from Example 2 was detected, the results are as follows:

**Table 11:**

| Animal | Administration route | T1/2(Hu049-18) (Day) | T1/2(Lilly mAb(hu)) (Day) |
|---|---|---|---|
| SD rat | IV (5 mg/kg) | 9.91 | 5.05 |
| | SC (5 mg/kg) | 11.5 | 5.53 |
| Macaca fascicularis | IV (1 mg/kg) | 24.4 | |

Conclusion: These results show that, compared to the control antibody of Lilly (T1/2 value of positive antibody in cynomolgus monkeys was reported as 6.5 days (iv) and 10.3 days (sc)), the antibody Hu049-18 of the present invention significantly prolonged the half-life in vivo under the described condition.

### Embodiments of the invention will now be described in the following numbered paragraphs:

1. An IL-17A binding agent, comprising:
   antibody light chain variable region, comprising 0-3 LCDR regions selected from those shown in SEQ ID NO: 13, SEQ ID NO: 14, and SEQ ID NO: 15; and
   antibody heavy chain variable region, comprising 0-3 HCDR regions selected from those shown in SEQ ID NO: 10, SEQ ID NO: 11, SEQ ID NO: 12;
   wherein the numbers of CDR regions of antibody light chain variable region and heavy chain variable regions are not simultaneously 0.
2. The IL-17A binding agent according to paragraph 1, which comprises one LCDR region selected from SEQ ID NO: 13, SEQ ID NO: 14 and SEQ ID NO: 15.
3. The IL-17A binding agent according to paragraph 1, which comprises one HCDR region selected from SEQ ID NO: 10, SEQ ID NO: 11 and SEQ ID NO: 12.
4. The IL-17A binding agent according to paragraph 1, which comprises two LCDR regions selected from SEQ ID NO: 13, SEQ ID NO: 14 and SEQ ID NO: 15.
5. The IL-17A binding agent according to paragraph 1, which comprises two HCDR regions selected from SEQ ID NO: 10, SEQ ID NO: 11 and SEQ ID NO: 12.
6. The IL-17A binding agent according to paragraph 1, which comprises three LCDR regions, wherein the amino acid sequence of LCDR1 is shown in SEQ ID NO: 13, the amino acid sequence of LCDR2 is shown in SEQ ID NO: 14, and the amino acid sequence of LCDR3 is shown in SEQ ID NO: 15.
7. The IL-17A binding agent according to paragraph 1, which comprises three HCDR regions, wherein the amino acid sequence of HCDR1 is shown in SEQ ID NO: 10, the amino acid sequence of HCDR2 is shown in SEQ ID NO: 11, and the amino acid sequence of HCDR3 is shown in SEQ ID NO: 12.
8. The IL-17A binding agent according to paragraph 1, wherein the antibody light chain variable region further comprises light chain FR region derived from murine κ chain or a variant thereof, or light chain FR region derived from murine λ-chain or a variant thereof.
9. The IL-17A binding agent according to paragraph 8, wherein the amino acid sequence of the antibody light chain variable region is shown in SEQ ID NO: 2.
10. The IL-17A binding agent according to paragraph 8, which further comprises light chain constant region derived from murine κ chain or a variant thereof, or light chain constant region derived from λ chain or a variant thereof.
11. The IL-17A binding agent according to paragraph 1, wherein the antibody heavy chain variable region further comprises heavy chain FR region derived from murine IgG1 or a variant thereof, heavy chain FR region derived from murine IgG2 or a variant thereof, heavy chain FR regions derived from murine IgG3 or a variant thereof, heavy chain FR regions derived from murine IgG4 or a variant thereof.
12. The IL-17A binding agent according to paragraph 11, wherein the amino acid sequence of antibody heavy chain variable region is shown in SEQ ID NO: 1.
13. The IL-17A binding agent according to paragraph 11, which further comprises heavy chain constant region derived from murine IgG1 or a variant thereof, heavy chain constant region derived from murine IgG2 or a variant thereof, heavy chain constant region derived from murine IgG3 or a variant thereof, heavy chain constant region derived from murine IgG4 or a variant thereof.
14. The IL-17A binding agent according to paragraph 1, wherein antibody light chain variable region further comprises light chain FR region derived from human κ chain or a variant thereof, or a light chain FR region derived from human λ chain or a variant thereof.
15. The IL-17A binding agent according to paragraph 14, wherein the light chain FR region is human germline light chain A10 FR region whose amino acid sequence is shown in SEQ ID NO: 4, or variants thereof.
16.The IL-17A binding agent according to paragraph 15, wherein the variant of human germline light chain A10 FR region refers to human germline light chain A10 FR region having 0-10 amino acid mutations.
17. The IL-17A binding agent according to paragraph 16, wherein the amino acid mutation is one or more selected from F71Y, K49Y, Y36F and L47W.
18. The IL-17A binding agent according to paragraph 14, wherein the antibody light chain is selected from light chain of SEQ ID NO: 9 or a variant thereof.
19. The IL-17A binding agent according to paragraph 14, which further comprises light chain constant region derived from human κ chain or a variant thereof, or light chain constant region derived from human λ chain or a variant thereof.
20. The IL-17A binding agent according to paragraph 1, wherein the heavy chain variable region further comprises heavy chain FR region derived from human IgG1 or a variant thereof, heavy chain FR region derived from human IgG2 or a variant thereof, heavy chain FR region derived from human IgG3 or a variant thereof, heavy chain FR region derived from human IgG4 or a variant thereof.
21. The IL-17A binding agent according to paragraph 20, wherein the heavy chain FR region is human germline heavy chain VH1-18 FR region whose amino acid sequence is shown in SEQ ID NO: 3, or a variant thereof.
22. The IL-17A binding agent according to paragraph 21, wherein the variant refers to heavy chain VH1-18 FR region having 0-10 amino acid mutations.
23. The IL-17A binding agent according to paragraph 22, wherein the amino acid mutation is one or more selected from A93T, T71A, M48I, V67A, M69L, T73D and S76N.
24. The IL-17A binding agent according to paragraph 21, wherein the heavy chain is selected from the heavy chains shown in SEQ ID NO: 5, SEQ ID NO: 6, SEQ ID NO: 7 and SEQ ID NO: 8, or a variant thereof.
25. The IL-17A binding agent according to paragraph 20, which further comprises heavy chain constant region derived from human IgG1 or a variant thereof, heavy chain constant region derived from human IgG2 or a variant thereof, heavy chain constant region derived from human IgG3 or a variant thereof, heavy chain constant region derived from human IgG4 or a variant thereof.
26. A vector expressing the IL-17A binding agent according to any one of the preceding numbered paragraphs.
27. The vector according to paragraph 26, which comprises nucleotide encoding the IL-17A binding agent according to any one of the preceding numbered paragraphs.
28. A pharmaceutical composition, comprising: the IL-17A binding agent according to any one of paragraphs 1-25; and a pharmaceutically acceptable excipient, diluent or carrier.
29. Use of the IL-17A binding agent according to any one of paragraphs 1-25, or the pharmaceutical composition according to paragraph 28, in the preparation of a medicament for treatment of IL-17 mediated diseases or disorders.
30. Use according to paragraph 29, wherein:
   the disease is selected from inflammatory and autoimmune diseases;
   the disease is preferably psoriasis, psoriatic arthritis, ankylosing spondylitis, multiple sclerosis, inflammatory arthritis.
31. A method for treating a disease or disorder mediated by IL-17, the method comprises administering to a subject in need thereof a therapeutically effective amount of the IL-17A binding agent according to any one of paragraphs 1-25, or the pharmaceutical composition according to paragraph 28.
32. The method for treating a disease or disorder mediated by IL-17 according to paragraph 31, wherein:
   the disease is selected from inflammatory and autoimmune diseases; the disease is preferably psoriasis, psoriatic arthritis, ankylosing spondylitis, multiple sclerosis, inflammatory arthritis.

## Claims

1. An IL-17A binding antibody or antigen-binding fragment thereof, comprising:
antibody light chain variable region, comprising LCDR1, LCDR2 and LCDR3 as shown in SEQ ID NO: 13, SEQ ID NO: 14, and SEQ ID NO: 15 respectively; and
antibody heavy chain variable region, comprising HCDR1, HCDR2 and HCDR3 as shown in SEQ ID NO: 10, SEQ ID NO: 11, and SEQ ID NO: 12, respectively.

2. The IL-17A binding antibody or antigen-binding fragment thereof according to claim 1, wherein the antibody light chain variable region further comprises light chain FR region derived from murine κ chain, or light chain FR region derived from murine λ-chain;
wherein the amino acid sequence of the antibody light chain variable region is shown in SEQ ID NO: 2.

3. The IL-17A binding antibody or antigen-binding fragment thereof according to claim 2, which further comprises light chain constant region derived from murine κ chain, or light chain constant region derived from λ chain.

4. The IL-17A binding antibody or antigen-binding fragment thereof according to claim 1, wherein the antibody heavy chain variable region further comprises heavy chain FR region derived from murine IgG1, heavy chain FR region derived from murine IgG2, heavy chain FR region derived from murine IgG3, or heavy chain FR region derived from murine IgG4;
wherein the amino acid sequence of antibody heavy chain variable region is shown in SEQ ID NO: 1.

5. The IL-17A binding antibody or antigen-binding fragment thereof according to claim 4, which further comprises heavy chain constant region derived from murine IgG1, heavy chain constant region derived from murine IgG2, heavy chain constant region derived from murine IgG3, or heavy chain constant region derived from murine IgG4.

6. The IL-17A binding antibody or antigen-binding fragment thereof according to claim 1, wherein antibody light chain variable region further comprises light chain FR region derived from human κ chain, or a light chain FR region derived from human λ chain;
wherein the light chain FR region is human germline light chain A10 FR region whose amino acid sequence is shown in SEQ ID NO: 4, or a variant thereof having 0-10 amino acid mutations;
wherein the amino acid mutation is one or more selected from F71Y, K49Y, Y36F and L47W, according to the Kabat numbering system.

7. The IL-17A binding antibody or antigen-binding fragment thereof according to claim 6, wherein the antibody light chain variable region is selected from light chain variable region of SEQ ID NO: 9.

8. The IL-17A binding antibody or antigen-binding fragment thereof according to claim 6 or 7, which further comprises light chain constant region derived from human κ chain, or light chain constant region derived from human λ chain.

9. The IL-17A binding antibody or antigen-binding fragment thereof according to claim 1, wherein the heavy chain variable region further comprises heavy chain FR region derived from human IgG1, heavy chain FR region derived from human IgG2, heavy chain FR region derived from human IgG3, or heavy chain FR region derived from human IgG4;
wherein the heavy chain FR region is human germline heavy chain VH1-18 FR region whose amino acid sequence is shown in SEQ ID NO: 3, or a variant thereof having 0-10 amino acid mutations;
wherein the amino acid mutation is one or more selected from A93T, T71A, M48I, V67A, M69L, T73D and S76N, according to the Kabat numbering system.

10. The IL-17A binding antibody or antigen-binding fragment thereof according to claim 9, wherein the heavy chain variable region is selected from the heavy chain variable regions shown in SEQ ID NO: 5, SEQ ID NO: 6, SEQ ID NO: 7 and SEQ ID NO: 8.

11. The IL-17A binding antibody or antigen-binding fragment thereof according to claim 9 or 10, which further comprises heavy chain constant region derived from human IgG1, heavy chain constant region derived from human IgG2, heavy chain constant region derived from human IgG3, or heavy chain constant region derived from human IgG4.

12. A vector expressing the IL-17A binding antibody or antigen-binding fragment thereof according to any one of the preceding claims.

13. The vector according to claim 12, which comprises nucleotide encoding the IL-17A binding antibody or antigen-binding fragment thereof according to any one of the preceding claims.

14. A pharmaceutical composition, comprising: the IL-17A binding antibody or antigen-binding fragment thereof according to any one of claims 1-11; and a pharmaceutically acceptable excipient, diluent or carrier.

15. The IL-17A binding antibody or antigen-binding fragment thereof according to any one of claims 1-11, or the pharmaceutical composition according to claim 14, for use in treatment of IL-17 mediated inflammatory or autoimmune diseases;
wherein the disease is selected from psoriasis, psoriatic arthritis, ankylosing spondylitis, multiple sclerosis or inflammatory arthritis.


Sequence

<110>  SHANGHAI HENGRUI PHARMACEUTICAL CO LTD, JIANGSU HENGRUI MEDICINE CO
LTD

<120>  IL-17A binding agent and uses thereof

<130>  GECBP/P60823EPdiv1

<150>  EP 14862443.0
<151>  2014-10-27

<150>  CN 20131580942
<151>  2013-11-18

<150>  PCT/CN2014/089542
<151>  2014-10-27

<160>  15

<170>  PatentIn version 3.3

<210>  1
<211>  123
<212>  PRT
<213>  mus musculus

<400>  1

His Val Gln Leu Gln Gln Ser Gly Ala Asp Leu Val Arg Pro Gly Ala
1               5                   10                  15

Ser Val Thr Leu Ser Cys Lys Ala Ser Gly Tyr Ile Phe Thr Asp Tyr
            20                  25                  30

Glu Val His Trp Val Lys Gln Thr Pro Val His Gly Leu Glu Trp Ile
        35                  40                  45

Gly Val Ile Asp Pro Gly Thr Gly Gly Val Ala Tyr Asn Gln Lys Phe
    50                  55                  60

Glu Gly Lys Ala Thr Leu Thr Ala Asp Asp Ser Ser Asn Thr Ala Tyr
65                  70                  75                  80

Met Glu Leu Arg Ser Leu Thr Ser Glu Asp Ser Ala Val Tyr Tyr Cys
                85                  90                  95

Thr Arg Tyr Ser Leu Phe Tyr Gly Ser Ser Pro Tyr Ala Met Asp Tyr
            100                 105                 110

Trp Gly Gln Gly Thr Ser Val Thr Val Ser Ser
        115                 120

<210>  2
<211>  106

<212>  PRT
<213>  mus musculus

<400>  2

Gln Ile Val Leu Thr Gln Ser Pro Ala Ile Met Ser Ala Ser Pro Gly
1               5                   10                  15

Glu Lys Val Thr Ile Thr Cys Ser Ala Ser Ser Ser Val Asn Tyr Met
            20                  25                  30

His Trp Phe Gln Gln Lys Pro Gly Thr Ser Pro Lys Leu Trp Ile Tyr
        35                  40                  45

Arg Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Arg Ser Ser Tyr Pro Trp Thr
                85                  90                  95

Phe Gly Gly Gly Thr Asn Leu Glu Ile Lys
            100                 105


<210>  3
<211>  98
<212>  PRT
<213>  homo sapiens VH1-18

<400>  3

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg

<210> 4
<211> 95
<212> PRT
<213> homo sapiens A10

<400> 4

Glu Ile Val Leu Thr Gln Ser Pro Asp Phe Gln Ser Val Thr Pro Lys
1               5                   10                  15

Glu Lys Val Thr Ile Thr Cys Arg Ala Ser Gln Ser Ile Gly Ser Ser
            20                  25                  30

Leu His Trp Tyr Gln Gln Lys Pro Asp Gln Ser Pro Lys Leu Leu Ile
        35                  40                  45

Lys Tyr Ala Ser Gln Ser Phe Ser Gly Val Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Thr Tyr Tyr Cys His Gln Ser Ser Ser Leu Pro
                85                  90                  95

<210> 5
<211> 123
<212> PRT
<213> Artificial

<220>
<223> humanized antibody mAb049

<400> 5

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Val His Trp Val Arg Gln Ala Pro Gly Gln Gly Leu Glu Trp Met
        35                  40                  45

Gly Val Ile Asp Pro Gly Thr Gly Gly Val Ala Tyr Asn Gln Lys Phe
    50                  55                  60

Glu Gly Arg Val Thr Met Thr Thr Asp Thr Ser Thr Ser Thr Ala Tyr

65                  70                  75                  80

Met Glu Leu Arg Ser Leu Arg Ser Asp Asp Thr Ala Val Tyr Tyr Cys
                85                  90                  95

Thr Arg Tyr Ser Leu Phe Tyr Gly Ser Ser Pro Tyr Ala Met Asp Tyr
            100                 105                 110

Trp Gly Gln Gly Thr Leu Val Thr Val Ser Ser
        115                 120

<210>  6
<211>  123
<212>  PRT
<213>  Artificial

<220>
<223>  humanized antibody mAb049

<400>  6

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Val His Trp Val Arg Gln Ala Pro Gly Gln Gly Leu Glu Trp Met
        35                  40                  45

Gly Val Ile Asp Pro Gly Thr Gly Gly Val Ala Tyr Asn Gln Lys Phe
    50                  55                  60

Glu Gly Arg Val Thr Met Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Thr Arg Tyr Ser Leu Phe Tyr Gly Ser Ser Pro Tyr Ala Met Asp Tyr
            100                 105                 110

Trp Gly Gln Gly Thr Leu Val Thr Val Ser Ser
        115                 120

<210>  7
<211>  123
<212>  PRT
<213>  Artificial

<220>

<223>  humanized antibody mAb049

<400>  7

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Val His Trp Val Arg Gln Ala Pro Gly Gln Gly Leu Glu Trp Ile
        35                  40                  45

Gly Val Ile Asp Pro Gly Thr Gly Gly Val Ala Tyr Asn Gln Lys Phe
    50                  55                  60

Glu Gly Arg Val Thr Met Thr Ala Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Thr Arg Tyr Ser Leu Phe Tyr Gly Ser Ser Pro Tyr Ala Met Asp Tyr
            100                 105                 110

Trp Gly Gln Gly Thr Leu Val Thr Val Ser Ser
        115                 120

<210>  8
<211>  123
<212>  PRT
<213>  Artificial

<220>
<223>  humanized antibody mAb049

<400>  8

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Val His Trp Val Arg Gln Ala Pro Gly Gln Gly Leu Glu Trp Ile
        35                  40                  45

Gly Val Ile Asp Pro Gly Thr Gly Gly Val Ala Tyr Asn Gln Lys Phe
    50                  55                  60

Glu Gly Arg Ala Thr Leu Thr Ala Asp Asp Ser Thr Asn Thr Ala Tyr

65                  70                  75                  80

Met Glu Leu Arg Ser Leu Arg Ser Asp Asp Thr Ala Val Tyr Tyr Cys
                85                  90                  95

Thr Arg Tyr Ser Leu Phe Tyr Gly Ser Ser Pro Tyr Ala Met Asp Tyr
            100                 105                 110

Trp Gly Gln Gly Thr Leu Val Thr Val Ser Ser
        115                 120

<210>  9
<211>  107
<212>  PRT
<213>  Artificial

<220>
<223>  humanized antibody mAb049

<400>  9

Glu Ile Val Leu Thr Gln Ser Pro Asp Phe Gln Ser Val Thr Pro Lys
1               5                   10                  15

Glu Lys Val Thr Ile Thr Cys Ser Ala Ser Ser Ser Val Asn Tyr Met
            20                  25                  30

His Trp Phe Gln Gln Lys Pro Asp Gln Ser Pro Lys Leu Trp Ile Tyr
        35                  40                  45

Arg Thr Ser Asn Leu Ala Ser Gly Val Pro Ser Arg Phe Ser Gly Ser
    50                  55                  60

Gly Ser Gly Thr Asp Tyr Thr Leu Thr Ile Asn Ser Leu Glu Ala Glu
65                  70                  75                  80

Asp Ala Ala Thr Tyr Tyr Cys Gln Gln Arg Ser Ser Tyr Pro Trp Thr
                85                  90                  95

Phe Gly Gln Gly Thr Lys Leu Glu Ile Lys Arg
            100                 105

<210>  10
<211>  5
<212>  PRT
<213>  mus musculus VH CDR1

<400>  10

Asp Tyr Glu Val His
1               5

<210>  11
<211>  17
<212>  PRT
<213>  mus musculus VH CDR2

<400>  11

Val Ile Asp Pro Gly Thr Gly Gly Val Ala Tyr Asn Gln Lys Phe Glu
1               5                   10                  15

Gly

<210>  12
<211>  14
<212>  PRT
<213>  mus musculus VH CDR3

<400>  12

Tyr Ser Leu Phe Tyr Gly Ser Ser Pro Tyr Ala Met Asp Tyr
1               5                   10

<210>  13
<211>  10
<212>  PRT
<213>  mus musculus VL CDR1

<400>  13

Ser Ala Ser Ser Ser Val Asn Tyr Met His
1               5                   10

<210>  14
<211>  7
<212>  PRT
<213>  mus musculus VL CDR2

<400>  14

Arg Thr Ser Asn Leu Ala Ser
1               5

<210>  15
<211>  9
<212>  PRT
<213>  mus musculus VL CDR3

<400>  15

Gln Gln Arg Ser Ser Tyr Pro Trp Thr
1               5